# EUROPEAN PATENT APPLICATION

(11) **EP 3 241 837 A1**
(43) Date of publication of application: **08.11.2017**
(21) Application number: 15874972.1
(22) Date of filing: 11.11.2015
(51) Int. Cl.: C07H 19/10, C07H 1/06, A61K 31/7072, A61P 31/14

(54) **METHOD FOR PREPARING SOFOSBUVIR CRYSTAL FORM-6**

(30) Priority: 29.12.2014 CN 201410837284
(71) Applicant: Zhejiang Hisun Pharmaceutical Co., Ltd, Zhejiang 318000 (CN)
(72) Inventor: GUO, Yanghui, Taizhou Zhejiang 318000 (CN); ZHAO, Shiguo, Taizhou Zhejiang 318000 (CN); ZHANG, Yue, Taizhou Zhejiang 318000 (CN); ZHANG, Liang, Taizhou Zhejiang 318000 (CN); YANG, Zhiqing, Taizhou Zhejiang 318000 (CN); CHAI, Jian, Taizhou Zhejiang 318000 (CN)
(74) Representative: HGF Limited
(86) International application number: PCT/CN2015/094264
(87) International publication number: WO 2016/107289

(57) **Abstract**

The present invention relates to a method for preparing Sofosbuvir crystal form-6. The method is simple in operation, stable in process condition, good in reproducibility, high in yield rate and high in purity, viscous gel-type products are not formed during the process, the problem of blocking of a discharge port is solved, and the method is suitable for mass industrial production and has high economic value.

## Description

### FIELD

The present invention is in the field of pharmaceutical chemistry, and in particular relates to a method for preparing Sofosbuvir crystal form-6.

### BACKGROUND

Sofosbuvir was a new generation of anti-hepatitis C virus drugs developed by the Gilead Sciences. Inc., US and was approved for marketing by the FDA in the United States in December 2013, under the trade name Sovaldi. The CAS registry number for Sofosbuvir was 1190307-88-0, and the chemical name thereof is: (S)-Isopropyl-2-(((S)-((2R,3R,4R,5R)-5-(2,4-dioxo-3,4-dihydropyrimidin-1(2H)-yl)-4-fluoro-3-h ydroxy-4-methyltetrahydrofuran-2-yl)methoxy)(phenoxy)phosphoryl)amino)propanoate, the formula is: C₂₂H₂₉FN₃O₉P, with the following structure:

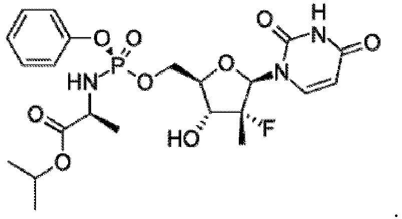

U.S. Patent US8618076 disclosed at least six crystalline forms of Sofosbuvir: Sofosbuvir crystal forms 1-6 and they were analyzed by using X-ray powder diffraction (XRPD), single crystal X-ray diffraction, differential scanning calorimetry (DSC) and so forth. Wherein, the Sofosbuvir crystal form-6 was characterized by the following X-ray powder diffraction 2θ characteristic peaks: 6.1°, 8.2°, 10.4°, 12.7°, 17.2°, 17.7°, 18.0°, 18.8°, 19.4°, 19.8°, 20.1°, 20.8°, 21.8°, 23.3°. Patent US8618076 also discloses a method for preparing Sofosbuvir crystal form-6, however, the method crystallizes Sofosbuvir in water, with a long crystallization process, difficult to control the process condition and poor in reproducibility, and crystal form-6 cannot be stably produced. In addition, a large amount of viscous gel-type products are formed during the process, easy to block the discharge port, and the method is not suitable for scale up production. Therefore, there is a need in the art to find a more practical method for preparing Sofosbuvir crystalline form-6.

### SUMMARY

The object of the present invention to provide a method for preparing Sofosbuvir crystal form-6 in order to overcome the drawbacks in the conventional method for preparing Sofosbuvir crystal form-6. The method has the advantages of simple in operation, stable in process condition, good in reproducibility, high in yield rate and good in purity, and has high economic value.

In order to solve the above-mentioned problems in the prior art, the present invention adopts the following technical solution.

In one aspect, the present invention provides a method for preparing Sofosbuvir crystal form-6, comprising the steps of:
(a) dissolving Sofosbuvir in an organic solvent to obtain a solution of Sofosbuvir;
(b) adding the solution obtained in step (a) to pure water preheated to a certain temperature;
(c) cooling the mixed solution obtained in step (b) to -15 to 25°C to obtain a Sofosbuvir crystal form-6.

In one embodiment of the present invention, the mixed solution obtained in step (b) is cooled to 0 to 10°C.

In another embodiment of the present invention, the method further comprises the step of incubating, stirring and crystallizing the mixed solution obtained in step (b) before step (c).

In still another embodiment of the present invention, the organic solvent in step (a) is selected from the group consisting of methanol, ethanol, isopropanol, acetonitrile, acetone, tetrahydrofuran, 1,4-dioxane, N,N-dimethylformamide, dimethylsulfoxide and mixtures thereof.

In a preferred embodiment of the present invention, the organic solvent in step (a) is selected from the group consisting of methanol, ethanol, acetone and mixtures thereof

In still another embodiment of the present invention, the volume/mass ratio (ml/g) of the organic solvent to the Sofosbuvir in step (a) is 1:1 to 10:1.

In a preferred embodiment of the present invention, the volume/mass ratio (ml/g) of the organic solvent to the Sofosbuvir in step (a) is 2:1 to 4:1.

In yet another embodiment of the present invention, the preheated temperature of the pure water in step (b) is 30 to 80°C.

In a preferred embodiment of the present invention, the preheated temperature of the pure water in step (b) is 40 to 60°C.

In yet another embodiment of the present invention, the volume ratio of pure water to organic solvent is 3:1 to 100:1.

In a preferred embodiment of the present invention, the volume ratio of pure water to organic solvent is 4:1 to 20:1.

According to another aspect of the present invention, the preparing method above further comprises the step of adding a seed of crystal form-6 to the mixed solution obtained in step (b).

In a preferred embodiment of the present invention, the mass ratio of the added seed to the Sofosbuvir is 1:1000 to 1:10.

In another preferred embodiment of the present invention, the mass ratio of the seed to the Sofosbuvir is 1:200 to 1:50.

The diffraction angles 2θ of the X-ray powder diffraction pattern of the Sofosbuvir crystal form-6 prepared by the above method have characteristic peaks at 6.1°, 8.2°, 10.4°, 12.7°, 17.2°, 17.7°, 18.0°, 18.8°, 19.4°, 19.8°, 20.1°, 20.8°, 21.8°, and 23.3°± 0.2.

In an embodiment of the present invention, the Sofosbuvir raw material used in the present invention can be prepared by using the methods disclosed in the prior art, such as, US8633309B.

### Advantages of the present invention:

The preparation method of Sofosbuvir crystal form-6 of the present invention is simple in operation and easy to implement, good in reproducibility, high in yield rate and high in purity, viscous gel-type products are not formed during the process, problem of blocking of a discharge is effectively solved, and it is very suitable for mass industrial production.

### BRIEF DESCRIPTION OF THE DRAWINGS

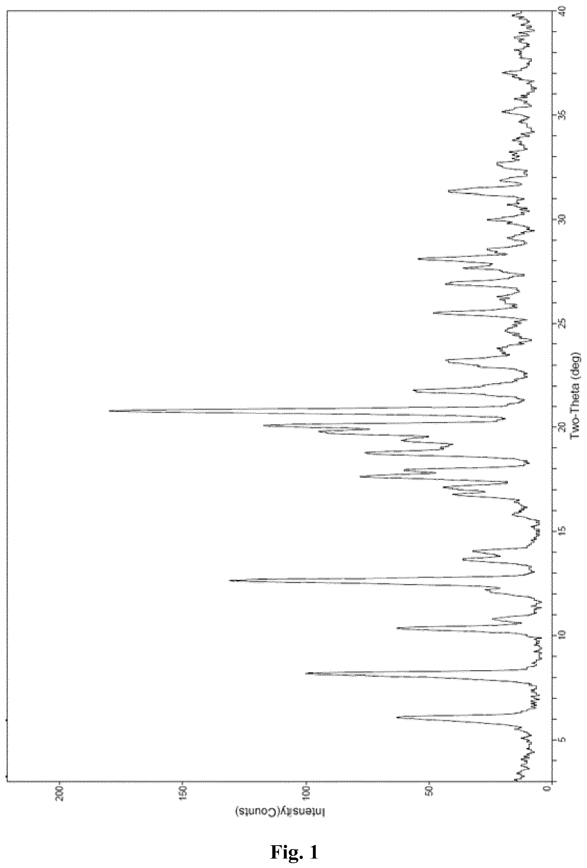

Figure 1: X-ray powder diffraction pattern of Sofosbuvir crystal form-6 prepared in Example 1.

### DETAILED DESCRIPTION

The present invention will be described below in detail by the specific examples, but should not be construed as limiting the invention in any way.

### Example 1

10 g of Sofosbuvir was dissolved in 100 ml of methanol, and the solution was added to 300 ml of pure water preheated to 45°C, after the completion of the addition, the mixed solution was cooled to -15°C, stirred for 2 to 3 hours and filtered. The filter cake was dried under vacuum to constant weight at 60°C to obtain 9.1 g of a white solid, and the yield rate was 91% with a purity of 99.7%. The 2θ angles of the X-ray powder diffraction pattern of the preparation product have characteristic peaks at 6.1°, 8.2°, 10.4°, 12.7°, 17.1°, 17.6°, 18.0°, 18.8°, 19.4°, 19.8°, 20.1°, 20.8°, 21.8°, 23.3°, it is confirmed as the Sofosbuvir crystal form-6.

### Example 2

10 g of Sofosbuvir was dissolved in 80 ml of ethanol, and the solution was slowly added dropwise to 480 ml of pure water preheated to 60°C, and 0.05 g of Sofosbuvir crystal form-6 was added as seeds. After the completion of addition, the mixed solution was incubated and stirred for about 3 to 4 hours for crystallization, and then it was cooled to 0°C, stirred for 2 to 3 hours and filtered. The filter cake was dried under vacuum to constant weight at 60°C to obtain 9.6 g of a white solid, and the yield rate was 96% with a purity of 99.6%. The X-ray powder diffraction pattern of the obtained product was consistent with the Sofosbuvir crystal form-6.

### Example 3

10 g of Sofosbuvir was dissolved in 60 ml of acetone, and the solution was slowly added dropwise to 480 ml of pure water preheated to 60°C, and 0.2 g of Sofosbuvir crystal form-6 was added as seeds. After the completion of addition, the mixed solution was incubated and stirred for about 3 to 4 hours for crystallization, and then it was cooled to 0°C, stirred for 2 to 3 hours and filtered. The filter cake was dried under vacuum to constant weight at 60°C to obtain 9.4 g of a white solid, and the yield rate was 94% with a purity of 99.5%. The X-ray powder diffraction pattern of the obtained product was consistent with the Sofosbuvir crystal form-6.

### Example 4

10 g of Sofosbuvir was dissolved in 10 ml of tetrahydrofuran, and the solution was slowly added dropwise to 1000 ml of pure water preheated to 50°C, and 1.0 g of Sofosbuvir crystal form-6 was added as seeds. After the completion of addition, the mixed solution was incubated and stirred for about 3 to 4 hours for crystallizztion, and then it was cooled to 5°C, stirred for 2 to 3 hours and filtered. The filter cake was dried under vacuum at 60°C to constant weight to obtain 9.6 g of a white solid, and the purity was 99.6%. The X-ray powder diffraction pattern of the obtained product was consistent with the Sofosbuvir crystal form-6.

### Example 5

10 g of Sofosbuvir was dissolved in 20 ml of N,N-dimethylformamide, and the solution was slowly added dropwise to 400 ml of pure water preheated to 30°C. After the completion of the addition, the mixed solution was incubated and stirred for about 3 to 4 hours for crystallization, and then it was cooled to 0°C, stirred for 2 to 3 hours, and filtered. The filter cake was dried under vacuum at 60°C to constant weight to obtain 9.5 g of white solid, and the yield rate was 95% with a purity of 99.5%. The X-ray powder diffraction pattern of the obtained product was consistent with the Sofosbuvir crystal form-6.

### Example 6

10 g of Sofosbuvir was dissolved in 20 ml of dimethylsulfoxide, and the solution was slowly added dropwise to 400 ml of pure water preheated to 80°C. After the completion of the addition, the mixed solution was incubated and stirred for about 3 to 4 hours for crystallization, and then it was cooled to 25°C, and stirred for 2 to 3 hours. The filter cake was dried under vacuum at 60°C to constant weight to obtain 9.5 g of a white solid, and the yield rate was 95% with a purity of 99.4%. The X-ray powder diffraction pattern of the obtained product was consistent with the Sofosbuvir crystal form-6.

### Example 7

10 g of Sofosbuvir was dissolved in 60 ml of acetonitrile, and the solution was slowly added dropwise to 480 ml of pure water preheated to 60°C, and 0.2 g of Sofosbuvir crystal form-6 was added as seeds. After the completion of the addition, the mixed solutuion was incubated and stirred for about 3 to 4 hours for crystallization, and then it was cooled to 0°C, stirred for 2 to 3 hours and filtered. The filter cake was dried under vacuum to constant weight at 60°C to obtain 9.4 g of a white solid, and the yield rate was 94% with a purity of 99.7%. The X-ray powder diffraction pattern of the obtained product was consistent with the Sofosbuvir crystal form-6.

### Example 8

1.0 kg of Sofosbuvir was dissolved in 4.0 liters of methanol, and the solution was slowly added dropwise to 32 liters of pure water preheated to 50°C and 1.0 g of Sofosbuvir crystal form-6 was added as seeds. After the completion of the addition, the mixed solution was incubated and stirred for about 3 to 4 hours for crystallization, and then it was cooled to 0°C, stirred for 2 to 3 hours and filtered. The filter cake was dried under vacuumat 60°C to constant weight to obtain 970 g of a white solid, and the yield rate was 97% with a purity of 99.6%. The X-ray powder diffraction pattern of the obtained product was consistent with that of the Sofosbuvir crystal form-6. The melting point of the prepared product was 124 degrees Celsius.

## Claims

1. A method for preparing Sofosbuvir crystalline form-6, comprising:
(a) dissolving Sofosbuvir in an organic solvent to obtain a solution of Sofosbuvir;
(b) adding the solution obtained in step (a) to pure water preheated to a certain temperature;
(c) cooling the mixed solution obtained in step (b) to -15 to 25°C, preferably 0 to 10°C, to obtain a Sofosbuvir crystal form-6.

2. The method according to claim 1, wherein further comprising the step of incubating, stirring and crystallizing the mixed solution obtained in step (b) before step (c).

3. The method according to claim 1 or 2, wherein the organic solvent in step (a) is selected from the group consisting of methanol, ethanol, isopropanol, acetonitrile, acetone, tetrahydrofuran, 1,4-dioxane, N,N-dimethylformamide, dimethylsulfoxide and mixtures thereof, preferably selected from the group consisting of methanol, ethanol, acetone and mixtures thereof.

4. The method according to any one of claims 1 to 3, wherein the volume/mass ratio (ml/g) of the organic solvent to the Sofosbuvir in step (a) is 1:1 to 10:1, preferably 2:1 to 4:1.

5. The method according to any one of claims 1 to 4, wherein the preheated temperature of the pure water in step (b) is 30 to 80°C, preferably 40 to 60°C.

6. The method according to any one of claims 1 to 5, wherein the volume ratio of the pure water to the organic solvent is 3:1 to 100:1, preferably 4:1 to 20:1.

7. The method according to any one of claims 1 to 6, wherein further comprising the step of adding a seed of the crystal form-6 to the mixed solution obtained in step (b).

8. The method according to claim 7, wherein the mass ratio of the added seed to the Sofosbuvir is 1:1000 to 1:10, preferably 1:200 to 1:50.

9. Use of Sofosbuvir crystalline form-6 obtained by the method according to any one of claims 1 to 7 in the manufacture of a medicament for treating hepatitis C virus.
